# EUROPÄISCHE PATENTANMELDUNG

(11) **EP 1 452 190 A1**
(43) Veröffentlichungstag der Anmeldung: **01.09.2004**
(21) Anmeldenummer: 03405132.6
(22) Anmeldetag: 28.02.2003
(51) Int. Cl.: A61L 27/34, A61L 27/38, C12N 5/00

(54) **Implantatkörper mit Multiblock-Copolymer- und Polylysin-Beschichtung**

(71) Anmelder: Eidgenössische Technische Hochschule (ETH), 8092 Zürich (CH); Universität Zürich, 8006 Zürich (CH)
(72) Erfinder: Neuenschwander, Peter, Dr.Ing.chem., 5400 Baden (CH); Zünd, Gregor, Dr.med., 8091 Zürich (CH); Hoerstrup, Simon, Philipp, Dr.med., 8091 Zürich (CH)
(74) Vertreter: Patentanwälte Schaad, Balass, Menzl & Partner AG

(57) **Zusammenfassung**

Die vorliegende Erfindung betrifft einen Implantatkörper mit einem formstabilen Träger, einer auf dessen Oberfläche angeordneten Multiblockcopolymerschicht und einer auf der Oberfläche des Multiblockcopolymers angeordneten Polylysinschicht. Das Trägermaterial enthält ein Polyglycolsäurepolymer oder eine Mischung eines Polyglycolsäurepolymers und ein weiteres biologisch abbaubares Polymer. Das Multiblockcopolymer enthält mindestens zwei chemisch verschiedene Blockbausteine, erhältlich durch lineare Polykondensation eines α,ω-Dihydroxypolyesters mit Diisocyanat, wobei der α,ω-Dihydroxypolyesters durch Transesterifikation von Poly-(R)-3-hydroxybuttersäure und/oder deren Copolymeren mit 3-Hydroxyvaleriansäure mit Ethylenglycol erhältlich ist, entweder mit einem weiteren α,ω-Dihydroxypolyester oder mit einem α,ω-Dihydroxypolyether, ausgewählt aus der Gruppe von α,ω-Dihydroxypoly(oxytetramethylen), α,ω-Dihydroxypoly(oxyethylen) und Copolymeren von Ethylenglycol und Propylenglycol.

## Beschreibung

Die vorliegende Erfindung betrifft neue Implantate und ein Verfahren zur Herstellung solcher Implantate, insbesondere solche in Form von Herzklappen oder Gefässe.

Aufgrund des allgemeinen Mangels an Organspendern, wurden bisher zur Behandlung einer Herzklappendysfunktion primär synthetisch hergestellte oder biologische Implantate tierischen Ursprungs eingesetzt.

Synthetische Implantate führen jedoch oft zu Fremdkörperreaktionen mit thromboembolytischen Komplikationen, die durch die mit der künstlichen Herzklappe veränderten Strömungsverhältnisse im Herzen begünstigt werden. Daher müssen die betroffenen Patienten lebenslänglich antikoaguliert werden, was eine erhöhte Blutungsgefahr zur Folge hat. Überdies sind Patienten mit synthetischen Herzklappen infektionsgefährdet, wobei die Infektionen oft lebensbedrohliche Komplikationen zur Folge haben.

Bei den biologischen Implantaten handelt es sich meistens um Schweineherzklappen oder um Klappen aus Rindergewebe, die mittels Glutaraldehyd behandelt und fixiert werden. Diese biologischen Implantate können mit guten Ergebnissen bei älteren Patienten eingesetzt werden, aber sie neigen zu Degeneration nach 12 bis 15 Jahren, so dass sie für jüngere Patienten in der Regel nicht in Betracht kommen. Überdies neigen sie zur Calcifizierung, so dass ihr Einsatz bei Kindern und jungen Menschen, die einen erhöhten Kalziumstoffwechsel aufweisen, problematisch ist. Weiterhin besteht im Vergleich zum gesunden Herzen eine erhöhte Infektionsgefahr und das potenzielle Risiko der Übertragung von spezisübergreifenden Viren. Die oben genannten biologischen Implantate stellen ein körperfremdes Gewebe dar, das mit einer gewissen Wahrscheinlichkeit vom körpereigenen Immunsystem als fremd erkannt wird und damit lebensbedrohliche Immunprozesse auslösen kann.

Ein weiterer Nachteil der bisher beschriebenen Arten von Herzklappenimplantaten ist, dass es sich um nicht lebende Strukturen handelt, so dass ihnen wichtige Eigenschaften wie die Fähigkeit zu Reparationsprozessen, zur Rekonfiguration oder zum Wachstum fehlen. Daraus folgt unter Anderem, dass bei sehr jungen Herzklappenpatienten bisher regelmässig eine Reoperation in Kauf genommen werden musste. Zusätzlich zu dem jeder Herzoperation inhärenten Risiko steigt jedoch bei jeder Reoperation das Letalitätsrisiko, da durch die vorangegangenen Operationen erhebliche Verwachsungen im Thoraxbereich auftreten.

Aufgrund dieser Probleme mit herkömmlichen Implantaten wird seit einiger Zeit versucht, künstliche Herzklappen oder andere Organe oder Gewebe durch "tissue engineering" herzustellen. Das "tissue engineering" befasst sich mit der Entwicklung biohybrider Implantate, die im Köper zu Gewebe oder ganzen Organsystemen heranwachsen. Die Technik des "tissue engineerings" beinhaltet die Bereitstellung eines geeigneten biokompatiblen, vorzugsweise biologisch abbaubaren Implantates, das vorzugsweise mit autologen Zellen, die vom Empfänger stammen, besiedelt wird. Durch die Verwendung autologer Zellen können die mit Fremdkörperreaktionen verbundenen Probleme umgangen werden. Das biokompatible Implantat stellt vorübergehend im Patient eine biomechanische Gerüststruktur bereit, in die Zellen, zunächst z.B. Bindegewebszellen, wie Fibroblasten, Myofibroblasten, Knorpelzellen oder Knochenzellen und später Endothelzellen einwachsen und eine Matrixkomponente bilden können. Typische extrazelluläre Matrixbestandteile sind Kollagen, Elastan und Glykosaminglykane. Nach der Bildung eines neuen funktionellen Gewebes wird der Träger vorzugsweise wieder im Körper abgebaut.

Als biokompatible Trägermaterialien für Herzklappen wurden bereits Polyglykolsäure (PGA), Polyhydrooktanoate (PHO) und Polyhydroxyalkanoate (PHA) eingesetzt. Beispiele dafür finden sich einer nicht veröffentlichten US-Patentanmeldung.

Diese Materialien können jedoch den sehr hohen Anforderungen betreffend der mechanischen Eigenschaften und der Biokompatibilität noch nicht ausreichend genügen. Aus PGA bestehende Implantatkörper weisen eine Oberfläche auf, die für den Haftungsprozess der Zellen nicht optimal ist, da für eine gleichmässige Haftung einer ausreichend hohen Anzahl Zellen und deren Versorgung mit dem Nährmedium eine hohe Porosität des tragenden Gerüsts und eine Verbindung der Poren untereinander (Permeabilität) essenziell ist. PHA ist thermoplastisch und gut formbar, weist jedoch ungenügende Elastizitätseigenschaften auf. PHO wird im Körper nur langsam abgebaut.

Aufgabe der vorliegenden Erfindung ist es daher, einen Implantatkörper bereitzustellen, der einerseits einfach und kostengünstig herzustellen ist und optimale Haftungseigenschaften für die besiedelnden Zellen aufweist, d.h. eine gute Elastizität, Porosität und Permeabilität.

Diese Aufgabe wird gelöst mit einem Implantatkörper gemäss Anspruch 1. Weitere vorteilhafte Ausführungsformen sind in den abhängigen Ansprüchen beansprucht.

Durch die Kombination der Polylysinschicht und der Multiblockcopolymerschicht weist der erfindungsgemässe Implantatkörper eine hervorragende Porosität und Permeabilität sowie Elastizität aus. Eine Zellschicht haftet ausserordentlich gut an der Polylysinschicht und die dreidimensionalen Anordnung der Zellschicht wird durch den Implantatkörper vorgegeben. Der erfindungsgemässe Implantatkörper ist ausgesprochen gut verträglich und die Abbauprodukte des Implantatkörpers sind ebenfalls gut verträglich. Die Oberflächeneigenschaften der Polylysinschicht führen der ausgesprochen guten Biokompatibilität des erfindungsgemässen Implantatkörpers.

Der Abbauzeitraum der Polymere liegt in der Grössenordnung von wenigen Wochen bis wenigen Monaten und kann durch die Beschichtung ausgesprochen gut gesteuert werden. Der Begriff "Abbau", wie hier verwendet, bedeutet, dass zum Ablauf der genannten Zeitspannen die Anwesenheit des Polymers mittels Gaschromatographie praktisch nicht mehr nachweisbar ist, d.h. weniger als 5% des ursprünglichen Polymers verbleiben im Körper. Geeignete Nachweismethoden sind im Stand der Technik bekannt und können dem speziell nachzuweisenden Polymer angepasst werden. Beispielsweise können getrocknete Gewebeproben mit einer Mischung von n-Butanol und konzentrierter HCl im Verhältnis 9:1, 2 Stunden lang bei 110° C umgesetzt werden. Nach Abkühlen der Probe und Waschen mit Wasser wird der organische Anteil der Reaktionsmischung mittels Gaschromatographie analysiert. Eichkurven können mit Hilfe geeigneter Referenzstandards erstellt werden.

Bei Herzklappenimplantaten sind insbesondere deren mechanischen Eigenschaften extrem wichtig, da sie durch die pulsierende Wirkung des Herzens ständig einem Druck ausgesetzt werden.

Wie oben erwähnt enthält der erfindungsgemässe Implantatkörper einen beschichteten Träger. Das Trägermaterial besteht im Wesentlichen aus einem reinen Polyglycolsäurepolymer (PGA) oder aus einer Mischung von Polyglycolsäurepolymer mit anderen biologisch abbaubaren Polymeren, wie z.B. Poly-4-hydroxybutyrat(P4HB). Der Begriff im Wesentlichen wie hier verwendet bedeutet, dass geringfügige Mengen, d.h. weniger als 10% anderer Substanzen, wie beispielsweise Substanzen, welche die Oberfläche in irgendeiner gewünschten Weise modifizieren, eingeschlossen sein können.

Die zwischen dem Träger und der Polylysinschicht liegende Multiblockcopolymerschicht ist aus zwei Arten von Makrodiolen als Blockbausteine aufgebaut, wobei vorzugsweise das eine Makrodiol im Blockcopolymer kristalline Bereiche und das andere Makrodiol amorphe Bereiche im Polymer bildet. Bei den Makrodiolen, die im Blockcopolymer kristalline Bereiche bilden, handelt es sich um solche, die kristallisierbare Verbindungen sind, und bei den Makrodiolen, die amorphe Bereiche bilden, kann es sich um kristallisierbare oder nicht kristallisierbare Verbindungen handeln. Durch eine entsprechende Auswahl von Makrodiolen und deren Kombination miteinander können die physikalischen, chemischen und biologischen Eigenschaften des resultierenden Multiblockcopolymers innerhalb eines breiten Spektrums eingestellt werden.

Das Multiblockcopolymer enthält mindestens zwei chemisch verschiedene Blockbausteine, erhältlich durch lineare Polykondensation eines α,ω-Dihydroxypolyesters mit Diisocyanat entweder mit einem weiteren α,ω-Dihydroxypolyester oder mit einem α,ω- Dihydroxypolyether, ausgewählt aus der Gruppe von α,ω-Dihydroxypoly(oxytetramethylen), α,ω-Dihydroxy(oxyethylen) und Copolymeren von Ethylenglycol und Propylenglycol. Der α,ω-Dihydroxypolyester ist erhältlich durch Transesterifikation von Poly- (R)-3-hydroxybuttersäure und/oder deren Copolymeren mit 3-Hydroxyvaleriansäure mit Ethylenglycol.

In einer bevorzugten Ausführungsform sind die α, ω-Dihydroxypolyester durch ringöffnende Polymerisation von zyklischen Estern und Laktonen, insbesondere von (L,L)-Dilaktid, (D,D)- Dilaktid, (D,L)-Dilaktid, Diglykolid, δ-(R)-Butyrolakton, β-(S)-Butyrolakton, β-rac-Butyrolakton und ε-Caprolacton und deren Gemische, in Gegenwart eines aliphatischen Diols oder Polyether-Diols oder PolyesterDiols erhältlich. In einer weiteren bevorzugten Ausführungsform sind die biokompatiblen Multicopolymere ausgewählt aus der Gruppe von

Poly[poly[α,ω-dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-dihydroxy-[oligo(glycolid-block-ε-caprolacton)-ethylenoligo(glycolid-block-ε-caprolacton)]-alt-2,2,4-trimethylhexamethylen-1,6-diisocyanat]] (Formel 1);

Poly[poly[{α,ω-dihydroxy-poly-3-(R)-hydroxybutyrat)-ethylen -poly(3-(R)-hydroxybutyrat)]-alt-2,2,4-trimethylhexamethylen-1,6-diisocyanat]-co-poly[α,ω-dihydroxy-[oligo(glycolid-block-ε-caprolacton)]-ethylenoligo(glykolid-block-s-caprolacton)-alt-2,2,4-trimethylhexamethylen-1,6-diisocyanat]] (Formel 2);

Poly[poly[α,ω)-dihydroxy-[oligo(glycolid-ran-ε-caprolacton)-alt-2,2,4-trimethylhexamethylen-1,6-diisocyanat]-co-poly[α,ω-dihydroxy-[oligo(glycolid-ran-ε-caprolacton)ethylen-oligo(glycolid-block-s-caprolacton)]-alt-2,2,4-trimethylhexamethylen-1,6-diisocyanat]] (Formel 3) ;

Poly[poly[α,ω-dihydroxy-[oligo(glycolid-block-ε-caprolacton)-ethylen-oligo(glycolid-block-ε-caprolacton)]-alt-2,2,4-trimethylhexamethylen-1,6-diisocyanat]-copoly[α,ω-dihydroxy-[oligo(glycolid-block-ε-caprolacton)-ethylen-oligo(glycolid-block-s-caprolacton)-alt-2,2,4-trimethylhexamethylen-1,6-diisocyanat]] (Formel 4);

Poly[poly[α,ω-dihydroxy-poly(3-(R)-hydroxybutyrat)-ethylen-poly(3-(R)-hydroxybutyrat)]-alt-2,6-diisocyanatcapronsäuremethylester]-co-poly[α,ω-dihydroxy-[oligo(ε-caprolacton)-ethylen-oligo(ε-caprolacton)]-alt-2,6-diisocyanatcapronsäuremethylester]-co-poly(10,11-dihydroxyundecanoat-alt-2,6-diisocyanatcapronsäuremethylester)].

Die Herstellung der oben genannten Multiblockpolymere ist in EP 0 696 605 A1 beschrieben.

Einige bevorzugte Diisocyanate für die Polykondensation sind Hexamethylendiisocyanat, 2,2,4-Tetramethylhexamethylendiisocyanat, Cyclohexyl-1,4-diisocyanat, Cyclohexyl-1,2-diisocyanat, Isophorondiisocyanat, Methylendicyclohexyldiisocyanat oder L-Lysindiisocyanatmethylester.

Die Beschichtung des Trägers kann auf eine im Stande der Technik bekannte Weise erfolgen. Beispielsweise kann der Träger in eine Lösung des Multiblockcopolymers in einem geeigneten organischen Lösungsmittel eingetaucht oder damit besprüht werden. Nach Verdampfen des Lösungsmittels wird eine durchgehende Schicht des Multiblockcopolymers auf dem Träger gebildet. Auf dieser Multiblockcopolymerschicht kann die Polylysinschicht analog dazu aufgebracht werden.

Die Dicke der Beschichtung kann den Anforderungen entsprechend variiert werden, indem z.B. die eingesetzte Menge oder Konzentration verändert werden oder mehrere Schichten übereinander aufgetragen werden. Durch die Schichtdicke kann ebenfalls die Geschwindigkeit der biologischen Abbaubarkeit verändert werden.

Zur Herstellung eines Implantats in Form einer Herzklappe oder eines Gefässes durch die eingangs erwähnte Technik des "tissue engineerings" und der Verwendung des erfindungsgemässen Implantatkörpers wird der Träger vorzugsweise in der Form des gewünschten Implantates, d.h. einer dreisegliger Herzklappe oder einer Röhre vorgeformt.

Der Implantatkörper weist vorzugsweise eine Polymerdichte von ca. 40 bis 120 mg/cm³ auf. Unterhalb von 40 mg/cm³ ist die Polymerschicht in der Regel zu labil, oberhalb von 120 mg/cm³ ist die Polymerschicht zu dicht, um ein Eindringen der gewünschten Zellen innerhalb eines vertretbaren Zeitraums zu ermöglichen. In bevorzugten Ausführungsformen beträgt die Dichte 50 bis 80 mg/cm³, besonders bevorzugt etwa 70 mg/cm³. Die Polymerfasern des Implantatkörpers können z.B. einen Durchmesser von 6 bis 20 µm haben, bevorzugt 10 bis 18 µm. Es sind jedoch auch Polymerschichten mit andern Faserstärken denkbar, die einerseits dem Implantatkörper ausreichende Stabilität verleihen und andererseits die Besiedlung und Durchdringung mit den gewünschten Zellen erlauben. Weiterhin können offenporige, (schwammartige), Polymerformen verwendet werden. Hier haben sich Porengrössen von 80 bis 240 µm als bevorzugt erwiesen. Die Poren können z.B. durch die Technik des so genannten "salt leaching" erzielt werden, die dem Fachmann bekannt sind.

Vor der Besiedlung des Implantatkörpers mit den gewünschten Zellen wird eine Sterilisation durchgeführt. Falls eine Sterilisation durch Hitze ungünstig ist, da bei der Sterilisationstemperatur entweder der Implantatkörper verformt wird oder darauf befindliche Proteine/Peptide irreversibel denaturiert werden, wird kaltes Ethenoxidgas als Sterilisationsmittel verwendet.

Die Zellen mit denen der Implantatkörper besiedelt werden soll, können beispielsweise durch eine Biopsie vom gesunden Gewebe des Patienten erhalten, in vitro mittels bekannter Zellkulturtechniken vermehrt und anschliessend mit dem Implantatkörper in einem geeigneten Wachstumsmedium in Kontakt gebracht werden. Alternativ können immunologisch kompatible Zellen anderer Spender oder Zellen von etablierten differenzierten Zelllinien oder Stammzelllinien verwendet werden.

Vorzugsweise wird der Implantatkörper zunächst mit einer Fibroblastenkultur bzw. Myofibroblastenkultur und später mit einer Endothelzellenkultur inkubiert.

Das bei der Inkubation verwendete Medium kann jedes handelsübliche Zellkulturmedium sein, bevorzugt wird DMEM verwendet, dem wahlweise 5 oder 10% fötales Kälberserum zugesetzt sein können. Gewünschtenfalls können geeignete biologisch aktive Moleküle, wie z.B. Wachstumsfaktoren oder Vitamine zugesetzt werden.

In einem allgemeinen Verfahren zur Herstellung eines Implantates in Form einer Herzklappe oder Gefässes durch "tissue engineering" wird
a) ein Träger aus Polyglycolsäurepolymer oder einer Mischung eines Polyglycolsäurepolymers und einem weiteren biologisch abbaubaren Polymer gebildet oder geformt,
b) der Träger aus Schritt a) mit dem biokompatiblen Multiblockcopolymer beschichtet,
c) die Multiblockcopolymerschicht aus Schritt b) mit Polylysin beschichtet,
d) eine Bindegewebsschicht durch Aufbringen und Besiedeln von Bindegewebszellen auf der Oberfläche der Polylysinschicht aus Schritt d hergestellt,
e) eine Endothelschicht durch Aufbringen und Besiedeln von Endothelzellen auf der Bindegewebsschicht aus Schritt d) hergestellt.

Vorzugsweise wird der mit Zellen besiedelte Implantatkörper, d.h. das Implantat, in den späteren Wachstumsphasen steigenden Durchflussraten und Drücken ausgesetzt, um die physiologischen Bedingungen im Körper nach Implantation zu simulieren und das neu gebildete Implantatsgewebe auf diese Bedingungen einzustellen. Dies kann beispielsweise in einer pulsatilen Flusskammer geschehen. Besonders bevorzugt erfolgt die Steigerung der Durchflussraten und Drücke auf zyklische Weise.

In einer bevorzugten Ausführungsform erfolgt die Besiedlung des Implantatkörpers und die Herstellung eines funktionellen Implantates in Form einer Herzklappe oder eines Gefässes analog zu dem in EP00108981.1 detailliert beschriebenen Verfahren. Dort werden auch Verfahren zur Auswahl und Gewinnung geeigneter Zellen offenbart.

### Beispiel 1:

Zur Herstellung eines Implantates in Form einer Herzklappe wird ein nichtgewebtes Polyglycolsäurepolymer (Faserdurchmesser: 12 bis 15 µm, Polymerdichte 70 mg / ml, Albani International Research, Mansfield, MA, (USA) verwendet. Das Polymer wird so zugeschnitten, dass es eine Röhre mit 19 mm Durchmesser bildet. In diesen Conduit werden drei dreieckige Segel eingenäht. Dieser Träger wird dann in eine Lösung von Poly[poly[α,ω-dihydroxy-poly(3-(R)-hydroxybutyrat)-ethylen-poly(3-(R)-hydroxybutyrat)]-alt-2,2,4-trimethylhexamethylen-1,6-diisocyanat]-copoly[α,ω)-dihydroxy-[oligo(glycolid-block-ε-caprolacton)-ethylen-oligo(glycolid-block-ε-caprolacton)]-alt-2,2,4-trimethylhexamethylen-1,6-diisocyanat]] in dem Lösungsmittel eingetaucht. Nach Verdampfung des Lösungsmittels wird der Vorgang gegebenenfalls wiederholt, bis die gewünschte Schichtdicke des Multiblockcopolymers erreicht ist. Der mit dem Multiblockcopolymer beschichtete Träger wird in eine Lösung von Polylysin eingetaucht. Nach Verdampfung des Lösungsmittels wird der Vorgang gegebenenfalls wiederholt, bis die gewünschte Schichtdicke des Polylysins erreicht wird. Dieser Implatatkörper kann zum Herstellen von dreisegligen Klappen, d.h. Pulmonal-, Aorten- und Tricuspidalklappen, verwendet werden. Für Mitralklappen werden zwei Segel eingenäht.

Auch ist die Beschichtung des PGA-Polymers mit dem Multiblockcopolymers und dem Polylysin und anschliessende Hitzeformung zu beispielsweise einer dreisegligen Herzklappe möglich.

### Beispiel 2:

Zur Herstellung eines Implantates in Form eines Gefässes wird entweder dasselbe PGA-Polymer wie in Beispiel 1 oder ein Mischpolymer aus 90% PGA und 10% P4HB verwendet. Das Polymer wird um einen Zylinder von 0,5 cm gewickelt und mit einer Überlappungszone von ca. 1 cm unter Hitzezufuhr (60 - 70° C) verschweisst. Auf diesen röhrenförmigen Träger wird auf die in Beispiel 1 beschriebene Weise Poly[poly[α,ω-dihydroxy-poly(α-(ω)-hydroxybutyrat)-ethylen-poly(3-(R)-hydroxybutyrat)]-alt-2,2,4-trimethylhexamethylen-1,6-diisocyanatl-co-poly[α,ω-dihydroxy-[oligo(glycolid-block-ε-caprolacton)-ethylen-oligo(glycolid-block-s-caprolacton)]-alt-2,2,4-trimethylhexamethylen-1,6-diisocyanat]] und das Polylysin aufgebracht. Durch die Wahl verschiedener Zylinderdurchmesser können Implantate in Form von Gefässen mit verschiedenen Innendurchmessern hergestellt werden.

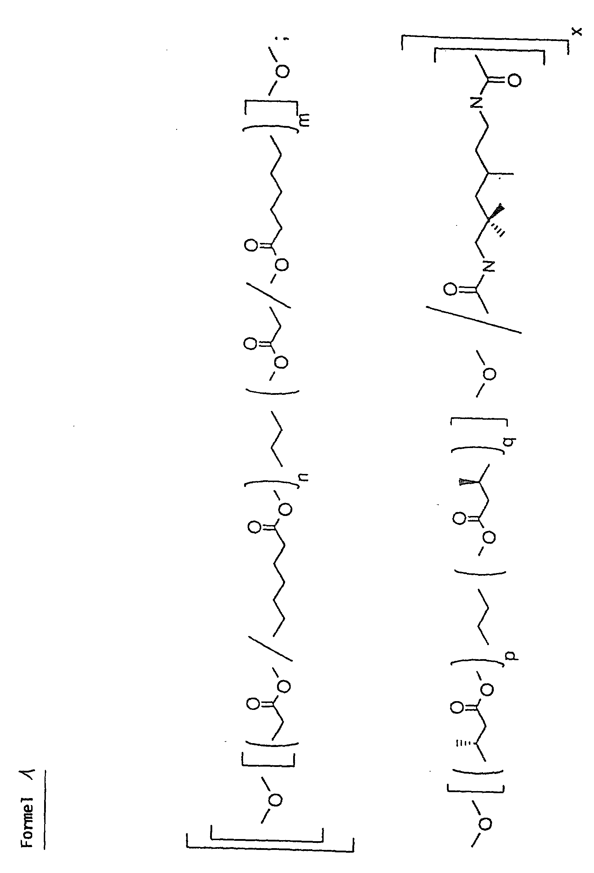

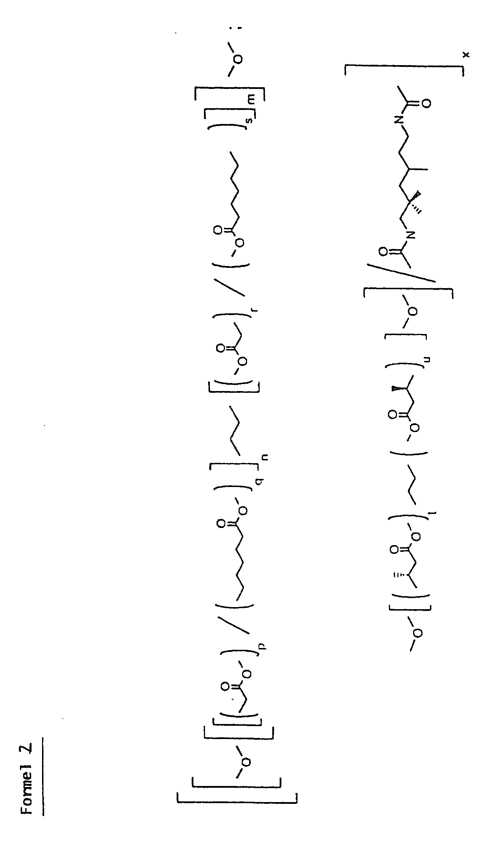

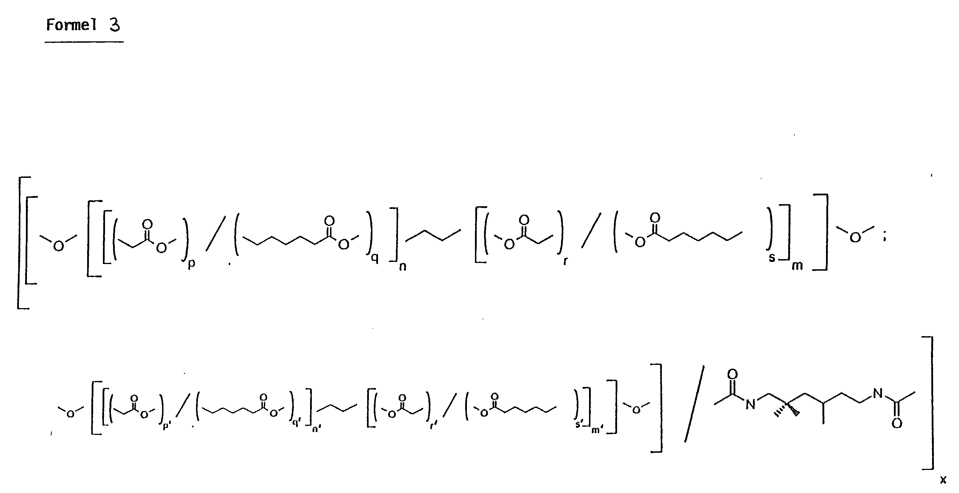

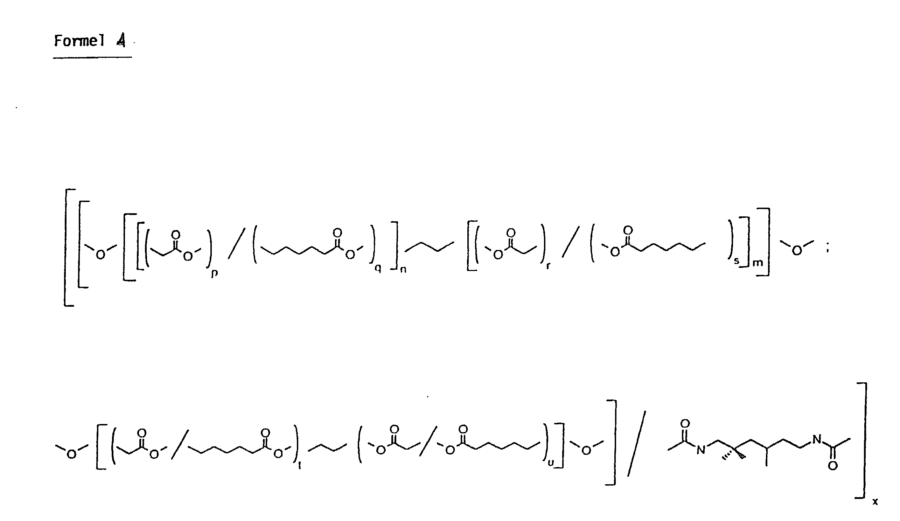

## Patentansprüche

1. Implantatkörper mit einem formstabilen Träger, einer auf dessen Oberfläche angeordneten Multiblockcopolymerschicht und einer auf der Oberfläche des Multiblockcopolymers angeordneten Polylysinschicht, wobei das Trägermaterial ein Polyglycolsäurepolymer oder eine Mischung eines Polyglycolsäurepolymers und einem weiteren biologisch abbaubaren Polymer enthält, und das Multiblockcopolymer mindestens zwei chemisch verschiedene Blockbausteine enthält, erhältlich ist durch lineare Polykondensation eines α,ω-Dihydroxypolyesters mit Diisocyanat, wobei der α,ω-Dihydroxypolyesters durch Transesterifikation von Poly-(R)-3-hydroxybuttersäure und/oder deren Copolymeren mit 3-Hydroxyvaleriansäure mit Ethylenglycol erhältlich ist, entweder mit einem weiteren α,ω-Dihydroxypolyester oder mit einem α,ω-Dihydroxypolyether, ausgewählt aus der Gruppe von α,ω-Dihydroxypoly(oxytetramethylen), α,ω-Dihydroxypoly(oxyethylen) und Copolymeren von Ethylenglycol und Propylenglycol.

2. Implantatkörper nach Anspruch 1 in Form einer Herzklappe.

3. Implantatkörper Anspruch 1 in Form eines Gefässes.

4. Implantat, enthaltend einen Implantatkörper nach einem der Ansprüche 1 bis 3, der mit Zellen besiedelt ist.

5. Verfahren zur Herstellung eines Implantats, in dem
a) ein Träger aus Polyglycolsäurepolymer oder einer Mischung eines Polyglycolsäurepolymers und einem weiteren biologisch abbaubaren Polymer gebildet oder geformt wird,
b) der Träger aus Schritt a) mit dem biokompatiblen Multiblockcopolymer nach einem der vorangehenden Ansprüche beschichtet wird,
c) die Multiblockcopolymerschicht aus Schritt b) mit Polylysin beschichtet wird,
d) eine Bindegewebsschicht durch Aufbringen und Besiedeln von Bindegewebszellen auf der Oberfläche der Polylysinschicht aus Schritt d hergestellt wird,
e) eine Endothelschicht durch Aufbringen und Besiedeln von Endothelzellen auf der Bindegewebsschicht aus Schritt d) hergestellt wird.

6. Verfahren nach Anspruch 5, wobei das in Schritt e erhaltene Implantat in einem fluiden Medium bei steigenden Flussraten und/oder Drücken ausgesetzt wird, um das Implantat an die physiologischen Bedingungen zu adaptieren, denen es in vivo ausgesetzt ist.

7. Verfahren nach Anspruch 6, indem das Implantat in einer pulsatilen Flusskammer zyklisch wiederkehrenden Druckveränderungen unterworfen wird.

8. Verfahren nach einem der Ansprüche 5, 6 oder 7, in dem die Bindegewebszellen Fibroblasten sind.

9. Implantat in Form einer Herzklappe oder eines Gefässes, herstellbar nach dem Verfahren nach einem der Ansprüche 5 bis 8.
